# EUROPÄISCHE PATENTANMELDUNG

(11) **EP 1 101 754 A2**
(43) Veröffentlichungstag der Anmeldung: **23.05.2001**
(21) Anmeldenummer: 00123617.3
(22) Anmeldetag: 28.10.2000
(51) Int. Cl.: C07C 51/353, C07C 57/15

(54) **Verfahren zur Reduktion der Menge an schwefelhältigen Nitrifikationshemmern**

(30) Priorität: 22.11.1999 AT 1907399
(71) Anmelder: DSM Fine Chemicals Austria Nfg GmbH & Co KG, 4021 Linz (AT)
(72) Erfinder: Zimmermann, Curt, 4310 Mauthausen (AT); Sengstschmid, Helmut, 4020 Linz (AT); Scheuchenstuhl, Willibald, 4271 Sankt Oswald/Freistadt (AT)
(74) Vertreter: Klostermann, Ingrid

(57) **Zusammenfassung**

Verfahren zur Reduktion der Menge an schwefelhältigen nitrifikationshemmenden Schwefelverbindungen in durch die mittels schwefelhältigen Verbindungen katalysierte Umwandlung von α-β-ungesättigten Carbonsäuren in das entsprechende trans-bzw. cis-Isomere erhaltenen Reaktionslösungen, bei welchem dem nach Beendigung der Isomerisierung erhaltenen Reaktionsgemisch ein Oxidationsmittel im molaren Unterschuss bis maximal in doppelter molarer Menge bezogen auf die Katalysatorverbindung zugesetzt wird und anschließend das entsprechende trans- bzw. cis-Isomere auf bekannte Weise isoliert und aufgereinigt wird und die entstehenden Prozessabwässer einer biologischen Aufreinigung zugeführt werden.

## Beschreibung

Die Erfindung betrifft ein Verfahren durch das die Restmenge an schwefelhältigen nitrifikationshemmenden Verbindungen, die als Isomerisierungskatalysator bei der Umwandlung von α-β-ungesättigten Carbonsäuren in das entsprechende trans-bzw. cis-Isomere eingesetzt werden, soweit reduziert wird, dass es bei der Aufarbeitung in biologischen Kläranlagen zu keiner Nitrifikationshemmung kommt.

Bei Prozessen im industriellen Maßstab fallen prinzipiell verschiedenste Abfälle an, welche ohne Umweltbelastung entsorgt werden müssen. Ein Problem bei der Entsorgung von Prozessabwässern stellt u.a. deren Belastung durch schwefelhältige Verbindungen dar. Thioharnsoff etwa wurde von der Environmental Protection Agency als gefährliche Substanz eingestuft. Er wirkt bekanntermaßen als Inhibitor beim Nitrifikationsprozeß in biologischen Kläranlagen. Insbesondere bei der Entfernung von Stickstoff und Phosphor ist die Nitrifikationsrate limitierend für die Leistung der biologischen Kläranlagen. Solche Kläranlagen reagieren daher sensibel auf spezielle, in Industrieabwässern vorhandene Inhibitoren. Da die meisten schwefelhältigen Verbindungen aber auch nitrifikationshemmend wirken, ist es somit erforderlich, den Gehalt dieser Verbindungen in den Prozessabwässer vor Einleiten in den Biokanal zu reduzieren.
Dazu können die nitrifikationshemmenden Verbindungen entweder vollständig entfernt oder zumindest deren Gehalt so weit reduziert werden, dass den Auflagen für eine umweltgerechte Entsorgung durch die biologischen Kläranlagen entsprochen wird. Unter schwefelhältigen Nitrifikationshemmern sind dabei neben Thioharnstoff Schwefelverbindungen, wie Thioamidverbindung, Thiocyanate, Thiazole oder Thiosemicarbazide zu verstehen, die vielfältig eingesetzt, u.a. bei der Herstellung und Modifizierung von Kunstharzen, Herstellung von Pharmazeutika, Industriereinigungsmitteln, Verwendung als Antioxidant und als Isomerisierungskatalysator bei der Umwandlung von α-β-ungesättigten Carbonsäuren in das entsprechende trans-bzw. cis-lsomere, werden.

Eine Entsorgungsmöglichkeit derart belasteter Abwässer stellt das Verbrennen, die thermische Zersetzung unter Verwendung von NaOH bei erhöhter Temperatur über einen längeren Zeitraum (z.B. 4 Tage) oder die Umsetzung mit Natriumhypochlorit dar. Die Nachteile dieser Verfahren sind einerseits die aufwendige Abgasreinigung (Denox und Entschwefelung) und dadurch verursachte zusätzliche Kosten, sowie Zeitverlust und andererseits, bei der Reaktion mit Natriumhypochlorit, das Entstehen von gefährlichen chlorierten organischen Verbindungen.
Als Verbesserung wird in US 4,822,494 ein zweistufiges Verfahren zur Entfernung von Thioharnstoff aus gebrauchten Salzsäure-Reinigungslösungen vorgeschlagen, bei welchem in der ersten Stufe die Lösung mit einem Alkali- oder Erdalkalihydroxid bis zum Erreichen eines pH-Wertes von mindestens 12 versetzt wird und in der zweiten Stufe zuerst mindestens 4 mol Wasserstoffperoxid pro mol Thioharnstoff und anschließend ein Alkali-oder Erdalkalihypochlorit zugegeben wird, wodurch der vorhandene Thioharnstoff zu Harnstoff oxidiert wird, der sodann vollständig zersetzt wird.
Dieses Verfahren ist jedoch ziemlich aufwendig und somit unwirtschaftlich.

Aufgabe der vorliegenden Erfindung war es daher ein einfaches Verfahren zu finden, durch das die Menge an schwefelhältigen nitrifikationshemmenden Verbindungen, die als Isomerisierungskatalysator bei der Umwandlung von α-β-ungesättigten Carbonsäuren in das entsprechende trans-bzw. cis-lsomere eingesetzt werden, soweit reduziert wird, dass eine umweltgerechte Entsorgung der Prozessabwässer gewährleistet ist.

Unerwarteterweise konnte diese Aufgabe durch einfache Zugabe eines Oxidationsmittels, im molaren Unterschuss bis maximal in doppelter molarer Menge, zu einer durch schwefelhältige Nitrifikationshemmer belasteten Lösung bzw. Reaktionslösung, erhalten durch Umwandlung von α-β-ungesättigten Carbonsäuren in das entsprechende trans-bzw. cis-lsomere, gelöst werden, wobei die Einstellung auf einen basischen pH-Wert entfällt und die Zugabe noch vor Isolierung von Reaktionsprodukten erfolgen kann.

Gegenstand der vorliegenden Erfindung ist demnach ein Verfahren zur Reduktion der Menge an schwefelhältigen nitrifikationshemmenden Schwefelverbindungen in durch die mittels schwefelhältigen Verbindungen katalysierte Umwandlung von α-β-ungesättigten Carbonsäuren in das entsprechende trans-bzw. cis-lsomere erhaltenen Reaktionslösungen, das dadurch gekennzeichnet ist, dass dem nach Beendigung der Isomerisierung erhaltenen Reaktionsgemisch ein Oxidationsmittel im molaren Unterschuss bis maximal in doppelter molarer Menge bezogen auf die Katalysatorverbindung zugesetzt wird und anschließend das entsprechende trans- bzw. cis-lsomere auf bekannte Weise isoliert und aufgereinigt wird und die entstehenden Prozessabwässer einer biologischen Aufreinigung zugeführt werden.

Erfindungsgemäß wird die Menge an schwefelhältigen nitrifikationshemmenden Verbindungen in durch schwefelhältigen Verbindungen katalysierte Umwandlung von α-β-ungesättigten Carbonsäuren in das entsprechende trans-bzw. cis-lsomere erhaltenen Reaktionslösungen reduziert, wodurch die bei dieser Reaktion anfallenden Prozessabwässern problemlos einer biologischen Aufarbeitung zugeführt werden können.

Unter schwefelhältigen Nitrifikationshemmern sind dabei Schwefelverbindungen, wie Thioamidverbindung, Thiocyanate, Thiazole oder Thiosemicarbazide zu verstehen. Bevorzugt sind Thioamidverbindungen.
Geeignete Thioamidverbindungen sind dabei Verbindungen der Formel 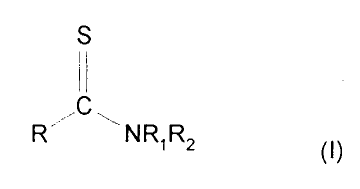 in der R H, C₁-C₂₀-Alkyl oder Alkenyl, Benzyl, Naphtyl oder NR₁R₂ bedeuten; R ₁und R₂ gleich oder verschieden sein können und H, oder C₁-C₁₀-Alkyl bedeuten.

Unter Alkyl- oder Alkenylreste sind dabei Reste zu verstehen, die linear, cyclisch oder verzweigt sein können, wie etwa Methyl, Ethyl, i-Propyl, n-Propyl, n-Butyl, t-Butyl, Hexyl, Dodecyl, Cyclopentyl, Cyclopentamethylen, u.s.w..
Beispiele dafür sind Thioformamid, Thioacetamid, Thiopropionamid, Thiobutyramid, Thiovaleramid, Thiododecylamid, Thiobenzamid, Thionaphthamid, Thioacrylamid, Thio-N-methyl-acetamid, Thio-N,N-dimethyl-acetamid, Thio-N,N-diethylacetamid, Thio-N-cyclo-pentamethylen-benzamid, Thioharnstoff, u.s.w..

Bevorzugt bedeutet R einen Rest NR₁R₂. Besonders bevorzugt bedeutet R einen Rest NR₁R₂ und R₁ und R₂ jeweils H. Besonders bevorzugte Thioamidverbindung ist demnach Thioharnstoff.

Das erfindungsgemäße Verfahren wird bei der Umwandlung von ungesättigten Verbindungen, wie etwa α-β-ungesättigten Carbonsäuren, in das entsprechende trans- bzw. cis-Isomere eingesetzt. Bevorzugt kommt das erfindungsgemäße Verfahren bei der Umwandlung von Maleinsäure in Fumarsäure zum Einsatz.

Bei der Aufarbeitung der durch oben angeführte Prozesse erhaltenen Reaktionslösungen, beispielsweise bei der Isolierung eines trans-Isomeren aus dem durch die als Katalysator verwendete Schwefelverbindung belasteten Reaktionsgemisch durch Abfiltrieren, gelangen diese nitrifikationshemmenden Schwefelverbindungen beispielsweise über die abgetrennte Mutterlauge in das Prozessabwasser.

Zur Reduzierung der Menge an schwefelhältigen Nitrifikationshemmern, wird dem durch einen der oben erwähnten Prozesse erhaltenen Reaktionsgemisch, bzw. Lösung, ein geeignetes Oxidationsmittel zugesetzt. Die Zugabe erfolgt bevorzugt direkt nach vollendeter Reaktion, noch vor der eigentlichen Aufarbeitung der Reaktionslösung. So wird beispielsweise bei der Herstellung von Fumarsäure aus Maleinsäure das Oxidationsmittel direkt nach Beendigung der Isomerisierung, noch vor der Isolierung der Fumarsäure, der Reaktionslösung zugesetzt.
Unerwarteterweise kommt es dabei zu keinen negativen Einflüssen auf den Reaktionsverlauf oder die Produktqualität. Weiters ist es nicht nötig, die Reaktionslösung alkalisch zu stellen und/oder die Temperatur des jeweiligen Reaktionsgemisches zu verändern.

Gegebenenfalls kann die Zugabe jedoch auch nach Aufarbeitung der Reaktionslösung bis kurz vor der biologischen Aufarbeitung des Prozessabwassers erfolgen.

Die Temperatur bei der Zugabe des Oxidationsmittels kann somit je nach Zeitpunkt der Zugabe zwischen Raumtemperatur und der Reaktionstemperatur des entsprechenden Prozesses liegen, wobei höhere Temperaturen die Reaktionskinetik positiv beeinflussen.

Geeignete Oxidationsmittel sind beispielsweise Ozon, anorganische lösliche Persulfate, Wasserstoffperoxid, organische Peroxide und Hydroperoxide, Persäuren, wie etwa Ammoniumpersulfat, Natriumpersulfat, Kaliumpersulfat, Lithiumpersulfat, Calciumpersulfat, Magnesiumpersulfat, Benzoylperoxid, Cyclohexanonperoxid, Acetylperoxid, Lauroylperoxid, t-Butylperoxid, t-Butylhydroperoxid, u.s.w..

Bevorzugt werden Wasserstoffperoxid oder anorganische Persulfate, besonders bevorzugt Wasserstoffperoxid als Oxidationsmittel verwendet.

Die Menge an zugesetztem Oxidationsmittel richtet sich nach dem gewünschten Grad der Reduzierung der Nitrifikationshemmung im Prozessabwasser. Die Nitrifikationshemmung, die im Prozessabwasser toleriert wird, kann von Land zu Land variieren. Je nach Auflage für die Kläranlagen, die die Prozessabwässer aufarbeiten, genügt es teilweise auch die Menge an schwefelhältigen Nitrifikationshemmern nur soweit zu reduzieren, dass zwar keine vollständige Beseitigung der Nitrifikationshemmung erzielt wird, aber Spitzenwerte, die nicht mehr toleriert werden, entsprechend gesenkt werden.

Aus diesem Grund wird das Oxidationsmittel je nach gewünschtem Grad der Reduzierung der Restmenge an schwefelhältigen Nitrifikationshemmern, im molaren Unterschuss bis zur doppelten molaren Menge bezüglich schwefelhältigen Nitrifikationshemmer zugegeben.
Bevorzugt wird das Oxidationsmittel in einer Menge von 0,1 mol bis 1mol pro mol schwefelhältigen Nitrifikationshemmer eingesetzt.

Durch die Zugabe des Oxidationsmittels werden die schwefelhältigen Nitrifikationshemmern soweit oxidiert, dass die Hemmung des Nitrifikationsprozesses in biologischen Käranlagen ausreichend bis vollständig beseitigt wird.

Besonders bevorzugt wird das Verfahren bei der Herstellung von Fumarsäure aus Maleinsäure unter Verwendung von Thioamidverbindungen der Formel (I) als Isomerisierungskatalysator angewandt. Unter den Thioamidverbindungen der Formel (I) ist wiederum Thioharnstoff besonders bevorzugt.

Das Oxidationsmittel, bevorzugt Wasserstoffperoxid oder anorganische Persulfate, besonders bevorzugt Wasserstoffperoxid, wird dem Reaktionsgemisch bevorzugt direkt nach Beendigung der Isomerisierungsreaktion zugegeben und das Reaktionsgemisch gerührt.
Die Temperatur bei der Zugabe liegt bevorzugt bei der Isomerisierungstemperatur, d.h. bei etwa 80-90°C. Gegebenenfalls kann das Reaktionsgemisch vor der Zugabe des Oxidationsmittels jedoch auch abgekühlt worden lassen.
Die bevorzugte Menge an Oxidationsmittel beträgt 0,1 bis 1 mol pro mol Katalysatorverbindung.

Die Rührzeit hängt von der Temperatur bei der Zugabe ab und liegt bevorzugt zwischen 1-120 Minuten, besonders bevorzugt zwischen 10 und 30 Minuten.

Es ist jedoch auch möglich, dass Oxidationsmittel erst später, nach der Abtrennung des trans-Isomeren, direkt in das Prozessabwasser zuzugeben. Die Zugabe kann dabei bis kurz vor der Aufarbeitung der Prozessabwässer in der Kläranlage erfolgen.

Durch das erfindungsgemäße Verfahren wird auf einfache und wirtschaftliche Weise eine Reduktion der Restmenge an schwefelhältigen Nitrifikationshemmern im Abwasser auf den vorgegebenen Wert erreicht. Die direkt Zugabe des Oxidationsmittels zu den jeweiligen Reaktionsgemischen bzw. -lösungen hat dabei sowohl auf den Reaktionsverlauf als auch auf die Produktqualität keinerlei negative Auswirkungen.

### Beispiel 1 (Standard)

In einem Reaktionsgefäß mit Rührer, Heizung, Thermometer und Rückflußkühler wurden 220 g (1,9 mol) Maleinsäure in Form einer 20%igen (g/g) wässrigen Lösung, welche bei einer Phthalsäureanhydrid-Produktion als Waschwasser angefallen war, gegeben. Neben Maleinsäure enthielt die Lösung noch 1%(g/g) Benzoesäure, 1,4%(g/g) Phthalsäure und kleine Mengen an nicht weiter untersuchten festen und gelösten teerartigen Bestandteilen. Der Anteil an Fumarsäure lag unter 0,2%.
Die Nitrifikationshemmung der Maleinsäurelösung wurde auf die übliche Weise durch Herstellung einer Verdünnungsreihe gemäß DIN EN 29888 gemessen. Das Ergebnis der Messung gibt die Verdünnung der Probe an, bei der eine festgelegte Hemmschwelle (EC 20) im Abbautest nicht mehr überschritten wird. Für die eingesetzte Maleinsäurelösung wurde ein Wert von 1:260 (EC20) gefunden.
Nach Erhitzen auf 90°C wurden 7,9 g (104 mmol) Thioharnstoff in Form einer 33%igen (g/g) heißen wäßrigen Lösung zugegeben und 2 h bei 90°C gerührt. Die so erhaltene Suspension wurde abgekühlt und filtriert. Die Bestimmung der Nitrifikationshemmung des Filtrats ergab einen Wert von 1:9110 (EC20).
Der Filterkuchen wurde in kochendem Wasser gelöst, mit Aktivkohle und Filterhilfsstoff versetzt und heiß filtriert. Das Filtrat wurde abgekühlt und der entstandene Niederschlag filtriert. Nach Trocknen des Filterkuchens wurden 192 g (1,653 mol) Fumarsäure erhalten (Ausbeute 87%).

### Beispiel 2 (unterstoichiometrische H₂O₂ Behandlung)

Analog zum Beispiel 1 wurden 220 g (1,9 mol) Maleinsäure in Form einer 20%igen (g/g) wäßrigen Lösung auf 90°C erhitzt. Wie im vorherigen Beispiel wurden 7,9 g (104 mmol) Thioharnstoff in Form einer 33%igen (g/g) heißen wäßrigen Lösung zugegeben und 2 h bei 90°C gerührt.

Danach wurden 5,9 g 30%iges (g/g) Wasserstoffperoxid (52 mmol) zugesetzt. Nach weiteren 15 min Rühren zeigte ein negativer Peroxidtest, dass das Wasserstoffperoxid vollständig mit Thioharnstoff reagiert hatte. Die Reaktionslösung wurde filtriert und die Nitrifikationshemmung des Filtrats analog Beispiel 1 bestimmt. Es wurde ein Wert von 1:3450 (EC20) gemessen.
Der Filterkuchen wurde wie im Beispiel 1 aufgearbeitet. Es wurden weder in der Ausbeute (192 g) noch in der Qualität der Fumarsäure (HPLC-Analyse und Peroxidtest) Unterschiede zum Beispiel 1 festgestellt.

### Beispiel 3 (stoichiometrische H₂O₂ Behandlung)

Analog zum Beispiel 1 wurden 220 g (1,9 mol) Maleinsäure in Form einer 20%igen (g/g) wäßrigen Lösung auf 90°C erhitzt. Dann 7,9 g (104 mmol) Thioharnstoff in Form einer 33%igen (g/g) heißen wäßrigen Lösung zugegeben und 2 h bei 90°C gerührt. Nach Zugabe von 11,8 g 30%igem (g/g) Wasserstoffperoxid (104 mmol H₂O₂) und weiteren 15 min Rühren zeigte ein negativer Peroxidtest, dass das Wasserstoffperoxid vollständig mit Thioharnstoff reagiert hatte. Die Reaktionslösung wurde filtriert und die Nitrifikationshemmung des Filtrats wie im Beispiel 1 bestimmt. Es wurde ein Wert von 1:680 (EC20) gefunden.
Der Filterkuchen wurde analog Beispiel 1 aufgearbeitet. Es wurden weder in der Ausbeute (192 g) noch in der Qualität der Fumarsäure (HPLC-Analyse und Peroxidtest) Unterschiede zum Beispiel 1 festgestellt.

## Patentansprüche

1. Verfahren zur Reduktion der Menge an schwefelhältigen nitrifikationshemmenden Schwefelverbindungen in durch die mittels schwefelhältigen Verbindungen katalysierte Umwandlung von α-β-ungesättigten Carbonsäuren in das entsprechende trans-bzw. cis-lsomere erhaltenen Reaktionslösungen, dadurch gekennzeichnet, dass dem nach Beendigung der Isomerisierung erhaltenen Reaktionsgemisch ein Oxidationsmittel im molaren Unterschuss bis maximal in doppelter molarer Menge bezogen auf die Katalysatorverbindung zugesetzt wird und anschließend das entsprechende trans- bzw. cis-lsomere auf bekannte Weise isoliert und aufgereinigt wird und die entstehenden Prozessabwässer einer biologischen Aufreinigung zugeführt werden.

2. Verfahren nach Anspruch 1, dadurch gekennzeichnet, dass als schwefelhältiger Nitrifikationshemmer eine Verbindung aus der Gruppe der Thioamidverbindungen, Thiocyanate, Thiazole oder Thiosemicarbazide in der Reaktionslösung vorliegt.

3. Verfahren nach Anspruch 2, dadurch gekennzeichnet, dass als schwefelhältiger Nitrifikationshemmer Thioamidverbindungen der Formel 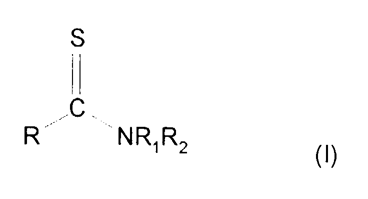 in der R H, C₁-C₂₀-Alkyl oder Alkenyl, Benzyl, Naphtyl oder NR₁R₂ bedeuten; R₁ und R₂ gleich oder verschieden sein können und H oder C₁-C₁₀-Alkyl bedeuten, vorliegen.

4. Verfahren nach Anspruch 1, dadurch gekennzeichnet, dass als Oxidationsmittel Ozon, anorganische lösliche Persulfate, Wasserstoffperoxid, organische Peroxide und Hydroperoxide oder Persäuren eingesetzt werden.

5. Verfahren nach Anspruch 4, dadurch gekennzeichnet, dass als Oxidationsmittel Wasserstoffperoxid oder anorganische Persulfate verwendet werden.

6. Verfahren nach Anspruch 1, dadurch gekennzeichnet, dass die durch schwefelhältige Verbindungen katalysierte Umwandlung von cis-α-β-ungesättigten Carbonsäuren in das entsprechende trans-Isomere die Herstellung von Fumarsäure aus Maleinsäure unter Verwendung von Thioamidverbindungen der Formel (I) als Isomerisierungskatalysator betrifft.

7. Verfahren nach Anspruch 1, dadurch gekennzeichnet, dass das Oxidationsmittel in einer Menge von 0,1 bis 1 mol pro mol Katalysatorverbindung eingesetzt wird.

8. Verfahren nach Anspruch 1, dadurch gekennzeichnet, dass das Oxidationsmittel dem Reaktionsgemisch direkt nach Beendigung der Isomerisierungsreaktion bei der Isomerisierungstemperatur zugegeben, das Reaktionsgemisch gerührt und anschließend das trans-bzw. cis-lsomere aus dem Reaktionsgemisch isoliert wird.
